# FASCICULE DE BREVET EUROPEEN

(11) **EP 3 743 006 B1**
(45) Date de publication et mention de la délivrance du brevet: **05.10.2022**
(21) Numéro de dépôt: 19701128.1
(22) Date de dépôt: 25.01.2019
(51) Int. Cl.: A61F 9/008, A61B 90/50, A61F 9/009, A61B 18/20, A61B 90/00, A61B 18/22, A61B 34/30

(54) **DISPOSITIF ET PROCEDE DE CONTROLE DU DEPLACEMENT D'UN APPAREIL DE THERAPIE OCULAIRE INCLUANT UN BRAS SUPPORT ARTICULE**
VORRICHTUNG UND VERFAHREN ZUR STEUERUNG DER BEWEGUNG EINER AUGENTHERAPIEVORRICHTUNG MIT EINEM GELENKIGEN STÜTZARM
DEVICE AND METHOD FOR CONTROLLING THE MOVEMENT OF AN OCULAR THERAPY APPARATUS INCLUDING AN ARTICULATED SUPPORT ARM

(30) Priorité: 25.01.2018 FR 1850578
(43) Date de publication de la demande: 02.12.2020
(73) Titulaire: Keranova, 42000 Saint Etienne (FR)
(72) Inventeur: BOULAROT, Nicolas, 42530 SAINT GENEST LERPT (FR); ROMANO, Fabrizio, 01700 BEYNOST (FR)
(74) Mandataire: Be IP
(86) Numéro de dépôt international: PCT/EP2019/051876
(87) Numéro de publication internationale: WO 2019/145487

(56) Documents cités:
- EP-A1- 3 266 403
- WO-A1-2017/182342

## Description

### DOMAINE TECHNIQUE

La présente invention concerne le domaine technique général du traitement de pathologies oculaires en utilisant un équipement de thérapie destiné à réaliser des opérations de l'œil, et plus particulièrement :
- des opérations du segment antérieur de l'œil comme la cataracte (au niveau du cristallin), et/ou
- des opérations de chirurgie réfractive (au niveau de la cornée), et/ou
- des opérations destinées à traiter le glaucome ou d'autres pathologies rétiniennes.

Plus précisément, l'invention concerne un dispositif et un procédé de contrôle du déplacement d'un système de traitement d'une pathologie oculaire monté sur un bras robotisé articulé pour permettre son déplacement selon trois axes orthogonaux X, Y et Z.

D'une manière générale, la présente invention trouve une application, lorsqu'un équipement de thérapie va agir dans l'œil en surface ou en profondeur au moyen d'agents physiques (tels que des ondes lumineuses, des ultrasons, des microondes, etc.), dont la trajectoire doit être précisément maîtrisée, afin d'atteindre la cible sans léser les structures adjacentes.

Dans la suite, on décrira l'invention en référence à un équipement de thérapie incluant un bras robotisé articulé intégrant un système de découpe d'un tissu humain ou animal, telle qu'une cornée, ou un cristallin, au moyen d'un laser femtoseconde.

Il est cependant bien évident pour l'homme du métier que l'invention décrite dans la suite peut être utilisée pour le contrôle du déplacement d'un bras robotisé articulé intégrant tout autre type de système de traitement d'une pathologie oculaire.

### ART ANTERIEUR

Il existe de nombreux équipements de thérapie incluant un laser pour le traitement d'une pathologie oculaire. Le laser est alors utilisé comme un bistouri optique.

Un tel laser est capable de réaliser des incisions des tissus transparents de l'œil, en profondeur, sans utiliser d'instruments chirurgicaux. Il présente l'avantage d'être rapide et bien toléré, mais surtout de supprimer le geste chirurgical manuel qui est opérateur dépendant.

Ainsi la chirurgie pratiquée avec un laser devient extrêmement précise et répétable. Elle apporte une garantie de sécurité qui ne peut être atteinte avec un geste pratiqué par un opérateur humain, de sorte que l'utilisation d'un laser permet d'envisager une chirurgie quasi-automatisée, où la machine va réaliser des étapes de la procédure chirurgicale à la place du praticien.

Pour qu'un équipement de thérapie incluant un laser puisse réaliser des étapes d'une procédure de traitement, il faut au préalable mettre en œuvre deux phases essentielles :
i) Fixer l'équipement de thérapie à l'œil, afin d'interdire les mouvements de l'œil pendant la procédure de traitement, et afin d'aligner l'axe de l'œil sur le référentiel de la machine ;
   de cette manière la machine et l'œil sont alignés et solidaires, et le traitement peut démarrer en toute sécurité, sans danger de déviation ou de mouvement en cours de procédure,
ii) Réaliser une cartographie des structures intraoculaires de l'œil du patient au moyen d'un système d'imagerie intégré de type OCT (tomographie par cohérence optique) ou Scheimpflug (cartographie en lumière visible), ou UBM (Ultrasonic Bio Microscopy) afin de tracer les contours des zones qui seront atteintes par le faisceau laser de manière à pouvoir les découper ou les fragmenter.

Pour réaliser l'étape i), il est nécessaire de positionner sur l'œil du patient un organe d'immobilisation équipé d'un anneau de succion capable de ventouser l'œil et de le maintenir fermement en position.

A l'heure actuelle, les équipements de thérapie agissant dans l'œil et nécessitant une immobilisation du globe oculaire pendant les phases i) et ii) (puis pendant la phase de traitement) sont tous équipés d'un organe d'immobilisation manipulé manuellement par l'opérateur.

De tels équipements de thérapie présentent de nombreux désavantages :
- la mise en place manuelle de l'organe d'immobilisation est soumise à une certaine variabilité, qui dépend de nombreux facteurs ; la qualité du positionnement de l'organe d'immobilisation varie notamment d'un opérateur à un autre ;
   ceci induit une variabilité des conditions d'immobilisation des patients, sachant que de la qualité du traitement est très dépendante de la qualité du positionnement de l'organe d'immobilisation,
- le temps de mise en place d'un organe d'immobilisation par l'opérateur (comme un chirurgien) est un temps très fortement valorisé et donc très coûteux, pour un geste qui pourrait être confié à une machine, qui le ferait de manière plus précise, répétable et pour un coût très inférieur,
- la manipulation de l'organe d'immobilisation est souvent pénible à effectuer, puisque l'opérateur n'est pas placé dans des conditions optimales et est souvent gêné par différents obstacles pour observer le globe oculaire et savoir si le positionnement de l'organe d'immobilisation est correct ou non,
- la faculté de l'opérateur à juger du bon positionnement de l'organe d'immobilisation oculaire, avis qui doit être basé sur des indications faisant appel à des repères dans l'espace (niveau de centrage, présence d'un tilt, d'une rotation, etc..) est très inférieure à celle d'une machine qui est équipée de capteurs et de systèmes d'imagerie capables de corriger les défauts de positionnement en X, Y ou Z ou en assiette, avec des niveaux de définition extrêmement précis,
- les patients peuvent, en fonction de la délicatesse de l'opérateur, subir un inconfort, une lésion, ou un mauvais positionnement de l'organe d'immobilisation susceptible de compromettre l'efficacité du traitement.

Le document EP 3 266 403 divulgue un dispositif de contrôle du déplacement d'un appareil de thérapie oculaire du type comprenant :
- un bras support, l'extrémité libre du bras étant destinée à venir au droit d'un tissu oculaire humain ou animal, ledit bras étant articulé pour permettre le déplacement de l'extrémité libre du bras selon trois axes X, Y et Z orthogonaux deux à deux :
   - l'axe X, définissant une direction longitudinale, horizontale,
   - l'axe Y, définissant une direction transversale, horizontale, qui avec l'axe X définit un plan XY horizontal,
   - l'axe Z, définissant une direction verticale, perpendiculaire au plan XY horizontal,
- un système d'acquisition monté sur le bras pour l'acquisition d'un couple de mesure comportant :
   - une image du tissu oculaire, et
   - un signal représentatif d'une distance verticale selon l'axe Z entre l'extrémité du bras et le tissu oculaire,
- des moyens de commande du système d'acquisition pour l'acquisition d'une pluralité de couples de mesure successivement dans le temps,
- des moyens de traitement de chaque couple de mesure, lesdits moyens de traitement incluant :
   - des moyens d'estimation, à partir du couple de mesure courant, de la distance verticale selon l'axe Z entre l'extrémité du bras et le tissu oculaire,
   - des moyens de calcul, à partir de l'image du couple de mesure courant, d'un écart horizontal entre : une position horizontale courante de l'extrémité libre du bras dans le plan horizontal XY, et une position horizontale finale désirée de l'extrémité libre du bras dans le plan horizontal XY.

Une ou deux valeurs seuil peuvent être établies pour contrôler le déplacement du bras jusqu'à la position et l`attitude finales.

Un but de la présente invention est de proposer un système intelligent et autonome, doté de mouvements robotisés, de vision, de capteurs et de facultés d'interprétation des images générées par la vision intégrée, pour automatiser la phase de mise en place de l'organe d'immobilisation du globe oculaire.

### EXPOSE DE L'INVENTION

A cet effet, l'invention concerne un dispositif de contrôle du déplacement d'un appareil de thérapie oculaire du type comprenant :
- un bras support, l'extrémité libre du bras étant destinée à venir au droit d'un tissu oculaire humain ou animal, ledit bras étant articulé pour permettre le déplacement de l'extrémité libre du bras selon trois axes X, Y et Z orthogonaux deux à deux :
   - l'axe X, définissant une direction longitudinale, horizontale,
   - l'axe Y, définissant une direction transversale, horizontale, qui avec l'axe X définit un plan XY horizontal,
   - l'axe Z, définissant une direction verticale, perpendiculaire au plan XY horizontal,
- un système d'acquisition monté sur le bras pour l'acquisition d'un couple de mesure comportant :
   - une image du tissu oculaire, et
   - un signal représentatif d'une distance verticale selon l'axe Z entre l'extrémité du bras et le tissu oculaire,
   ***remarquable en ce que*** le dispositif de contrôle comprend :
   - des moyens de commande du système d'acquisition pour l'acquisition d'une pluralité de couples de mesure successivement dans le temps,
   - des moyens de traitement de chaque couple de mesure, lesdits moyens de traitement incluant :
      - des moyens d'estimation, à partir du couple de mesure courant, de la distance verticale selon l'axe Z entre l'extrémité du bras et le tissu oculaire,
      - des moyens de calcul, à partir de l'image du couple de mesure courant, d'un écart horizontal entre :
         ▪ une position horizontale courante de l'extrémité libre du bras dans le plan horizontal XY, et
         ▪ une position horizontale finale désirée de l'extrémité libre du bras dans le plan horizontal XY,
   - des moyens d'asservissement pour :
      - si l'écart horizontal calculé est supérieur à une première valeur seuil, générer une consigne de déplacement horizontal du bras dans le plan horizontal XY pour réduire l'écart entre la position horizontale courante et la position horizontale finale désirée,
      - si l'écart horizontal calculé est inférieur à la première valeur seuil et si la distance verticale estimée est supérieure à une deuxième valeur seuil, générer une consigne de déplacement vertical du bras selon une direction verticale pour réduire la distance entre l'extrémité libre du bras et le tissu oculaire,
      - si l'écart horizontal calculé est inférieur à la première valeur seuil et si la distance verticale mesurée est inférieure à la deuxième valeur seuil, générer une consigne d'immobilisation du bras.

Ainsi, l'invention permet de rendre la phase de mise en place de l'équipement de thérapie plus précise, répétable et pour un coût inférieur aux solutions existantes.

Des aspects préférés mais non limitatifs du dispositif de contrôle sont les suivants :
- les moyens de calcul peuvent comprendre :
   - des moyens de détection, à partir de l'image du couple de mesure courant, de la position horizontale d'au moins un point d'intérêt du tissu oculaire,
   - des moyens d'évaluation, à partir de la position horizontale détectée du point d'intérêt, d'un écart horizontal entre :
      ▪ la position horizontale courante de l'extrémité libre du bras dans le plan horizontal XY, et
      ▪ la position horizontale finale désirée de l'extrémité libre du bras dans le plan horizontal XY ;
- les moyens de détection peuvent être aptes à identifier le tissu oculaire dans l'image acquise, par la mise en œuvre d'un algorithme de reconnaissance de forme pour détecter trois cercles concentriques dans l'image ;
- l'appareil de thérapie peut comprendre en outre un capteur de force monté sur l'extrémité libre du bras pour mesurer une force mécanique appliquée sur l'extrémité libre du bras :
   - les moyens de traitement comprenant des moyens de comparaison de ladite force mécanique mesurée à une troisième valeur seuil pour déterminer si l'extrémité libre du bras est en contact avec un élément faisant obstacle à un déplacement vertical du bras selon l'axe Z,
   - les moyens d'asservissement étant programmés pour générer une consigne d'immobilisation du bras si la force mécanique mesurée est supérieure à la troisième valeur seuil ;
- le système d'acquisition peut comprendre, pour l'acquisition d'un signal représentatif d'une distance verticale selon l'axe Z :
   - des moyens d'acquisition par télémétrie laser, et/ou
   - des moyens d'acquisition par ultrasons
   - des moyens d'acquisition par traitement d'image ;
- les moyens d'asservissement peuvent être programmés pour générer des consignes de déplacement élémentaires pour permettre le déplacement du bras entre sa position courante et une position finale désirée, lesdits moyens d'asservissement générant une consigne d'immobilisation consécutive à chaque consigne de déplacement élémentaire.

L'invention concerne également un procédé de contrôle du déplacement d'un appareil de thérapie oculaire du type comprenant :
- un bras support, l'extrémité libre du bras étant destinée à venir au droit d'un tissu oculaire humain ou animal, ledit bras étant articulé pour permettre le déplacement de l'extrémité libre du bras selon trois axes X, Y et Z orthogonaux deux à deux :
   - l'axe X, définissant une direction longitudinale, horizontale,
   - l'axe Y, définissant une direction transversale, horizontale, qui avec l'axe X définit un plan XY horizontal,
   - l'axe Z, définissant une direction verticale, perpendiculaire au plan XY horizontal ;
- un système d'acquisition monté sur le bras pour l'acquisition d'un couple de mesure comportant :
   - une image du tissu oculaire, et
   - un signal représentatif d'une distance verticale selon l'axe Z entre l'extrémité du bras et le tissu oculaire,
   ***remarquable en ce que*** le procédé de contrôle comprend les phases suivantes :
   - acquérir une pluralité de couples de mesure successivement dans le temps grâce au système d'acquisition,
   - traiter chaque couple de mesure, la phase de traitement comprenant les étapes consistant en :
      - l'estimation à partir du couple de mesure courant, de la distance verticale selon l'axe Z entre l'extrémité du bras et le tissu oculaire,
      - le calcul à partir de l'image du couple de mesure courant, d'un écart horizontal entre :
         ▪ une position horizontale courante de l'extrémité libre du bras dans le plan horizontal XY, et
         ▪ une position horizontale finale désirée de l'extrémité libre du bras dans le plan horizontal XY,
   - asservir le déplacement du bras en :
      - si l'écart horizontal calculé est supérieur à une première valeur seuil, générant une consigne de déplacement horizontal du bras dans le plan horizontal XY pour réduire l'écart entre la position horizontale courante et la position horizontale finale désirée,
      - si l'écart horizontal calculé est inférieur à la première valeur seuil et si la distance verticale estimée est supérieure à une deuxième valeur seuil, générant une consigne de déplacement vertical du bras selon une direction verticale pour réduire la distance entre l'extrémité libre du bras et le tissu oculaire,
      - si l'écart horizontal calculé est inférieur à la première valeur seuil et si la distance verticale mesurée est inférieure à la deuxième valeur seuil, générant une consigne d'immobilisation du bras.

Des aspects préférés mais non limitatifs du procédé de contrôle sont les suivants :
- l'étape de calcul peut comporter les sous-étapes suivantes :
   - la détection, à partir de l'image du couple de mesure courant, de la position horizontale d'au moins un point d'intérêt du tissu oculaire,
   - l'évaluation, à partir de la position horizontale détectée du point d'intérêt, d'un écart horizontal entre :
      ▪ la position horizontale courante de l'extrémité libre du bras dans le plan horizontal XY, et
      ▪ la position horizontale finale désirée de l'extrémité libre du bras dans le plan horizontal XY ;
- la sous-étape de détection peut consister à identifier le tissu oculaire dans l'image acquise, par la mise en œuvre d'un algorithme de reconnaissance de forme pour détecter trois cercles concentriques dans l'image ;
- l'appareil de thérapie peut comprendre en outre un capteur de force monté sur l'extrémité libre du bras pour mesurer une force mécanique appliquée sur l'extrémité libre du bras :
   - la phase de traitement comprenant une étape de comparaison de ladite force mécanique mesurée à une troisième valeur seuil pour déterminer si l'extrémité libre du bras est en contact avec un élément faisant obstacle à un déplacement vertical du bras selon l'axe Z,
   - l'étape d'asservissement incluant la génération d'une consigne d'immobilisation du bras si la force mécanique mesurée est supérieure à la troisième valeur seuil ;
- la phase consistant à acquérir peut comprendre :
   - l'acquisition par télémétrie laser d'un signal représentatif d'une distance verticale selon l'axe Z, et/ou
   - l'acquisition par ultrasons d'un signal représentatif d'une distance verticale selon l'axe Z, et/ou,
   - l'extraction d'une image acquise d'un signal représentatif d'une distance verticale selon l'axe Z ;
- l'étape d'asservissement peut comporter :
   - générer une consigne de déplacement élémentaire pour permettre le déplacement du bras entre sa position courante et une position finale désirée,
   - générer une consigne d'immobilisation consécutive à chaque consigne de déplacement élémentaire,
   - répéter les sous-étapes précédentes jusqu'à ce que l'écart horizontal calculé soit inférieur à la première valeur seuil et que la distance verticale mesurée soit inférieure à la deuxième valeur seuil.

### BREVE DESCRIPTION DES DESSINS

D'autres caractéristiques et avantages de l'invention ressortiront clairement de la description qui va suivre de plusieurs variantes d'exécution, données à titre d'exemples non limitatifs, à partir des dessins annexés sur lesquels :
- les figures 1 et 2 illustrent un appareil de thérapie incluant un bras support et un dispositif de contrôle selon l'invention, le bras étant :
   - dans une position rétractée sur la figure 1, et
   - dans une position déployée sur la figure 2,
- la figure 3 illustre schématiquement un système de découpe intégré à l'appareil de thérapie,
- la figure 4 illustre schématiquement les étapes d'un procédé de contrôle mis en œuvre dans le dispositif de contrôle,
- la figure 5 illustre schématiquement les étapes de déplacement du bras au cours d'une procédure de traitement d'une pathologie oculaire.

### EXPOSE DÉTAILLÉ DE L'INVENTION

L'invention concerne un dispositif et un procédé de contrôle du déplacement d'un appareil de thérapie d'un tissu oculaire humain ou animal. Dans la suite de la description, l'invention sera décrite, à titre d'exemple, pour la découpe d'un tissu oculaire, étant bien entendu que la présente invention peut être utilisée pour tout autre type de traitement oculaire.

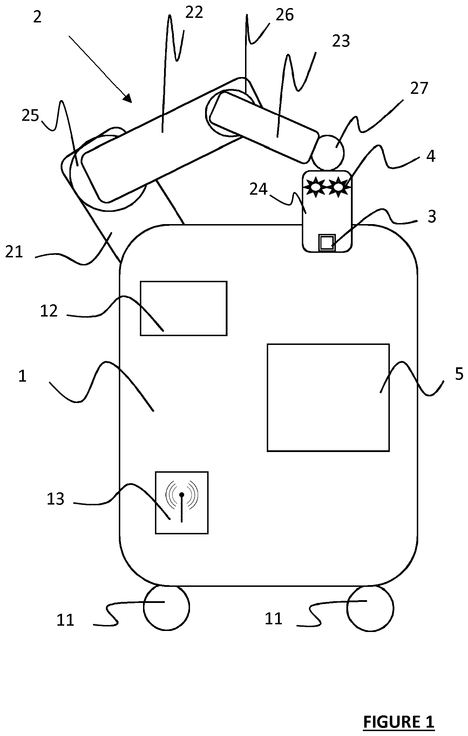

En référence à la figure 1, on a illustré un exemple d'appareil de thérapie.

L'appareil de thérapie comprend :
- un caisson mobile 1,
- un bras support articulé 2 monté sur le caisson 1,
- un système de découpe monté sur le bras 2,
- un capteur de forces 3 monté à une extrémité libre du bras 2,
- un système d'acquisition 4 monté sur le bras 2 pour l'acquisition d'images et de signaux représentatifs d'une distance entre l'extrémité libre du bras 2 et le tissu oculaire,
- un dispositif de contrôle 5 intégré au caisson 1, le dispositif de contrôle 5 incluant des moyens de commande et des moyens de traitement.

### 1. Caisson mobile

Le caisson 1 permet le déplacement de l'équipement de thérapie. Il comprend notamment des roues 11, un châssis métallique et un carénage approprié de manière à présenter un minimum de recoins afin d'éviter que des poussières ou des éléments pathogènes puissent s'y loger et prospérer.

Le caisson 1 comprend de préférence des moyens d'immobilisation par rapport au sol pour interdire son déplacement en cours d'intervention chirurgicale.

Le caisson 1 porte les différents éléments de l'équipement de thérapie - tels que le bras 2 et le dispositif de contrôle 5 - et comprend des moyens pour l'alimentation en énergie électrique de ceux-ci.

Le caisson 1 peut de plus comprendre des moyens d'affichage et de saisie 12 - tels qu'une console de planification - permettant au praticien de commander l'équipement de thérapie et/ou de suivre l'évolution du traitement appliqué à l'œil du patient.

Enfin, le caisson 1 peut comporter des moyens de communication 13 avec ou sans fil pour l'échange de données avec une station de travail distante (non représenté), ou avec le dispositif de contrôle 5 si celui-ci n'est pas intégré au caisson 1.

### 2. Bras support

Le bras 2 comprend plusieurs segments de bras 21-24 reliés par des articulations 25-27 (liaisons pivot ou rotule) pour permettre le déplacement en rotation des différents segments 21-24 les uns par rapport aux autres.

Chaque articulation 25-27 comporte une motorisation et un frein. Avantageusement, chaque frein est du type actif en cas d'absence d'alimentation en énergie électrique. Ceci permet d'interdire tout mouvement inopiné du bras, par exemple en cas de défaillance du système ou de coupure de courant.

Les motorisations et freins des articulations du bras permettent :
- un déplacement automatique des segments de bras 21-24 relativement au caisson 1, et
- l'immobilisation des segments de bras 21-24 relativement au caisson 1.

En particulier, le bras est articulé pour permettre le déplacement de l'extrémité libre du bras selon trois axes orthogonaux X, Y et Z :
- l'axe X, définissant une direction longitudinale, horizontale,
- l'axe Y, définissant une direction transversale, horizontale, qui avec l'axe X définit un plan XY horizontal,
- l'axe Z, définissant une direction verticale, perpendiculaire au plan XY horizontal.

L'extrémité libre du bras 2 comporte un organe d'immobilisation équipé d'un anneau de succion capable de ventouser le tissu oculaire et de le maintenir fermement en position. Le dispositif et le procédé de contrôle décrits dans la suite permettent de positionner automatiquement l'organe d'immobilisation sur le tissu oculaire à traiter.

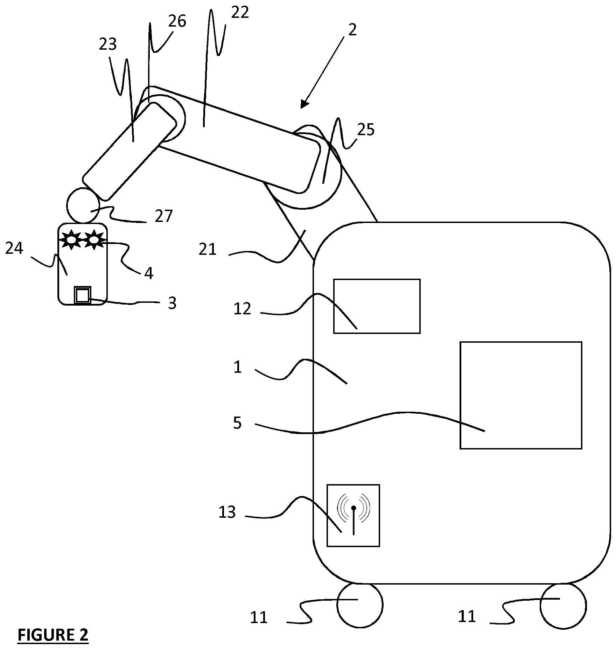

Comme illustré aux figures 1 et 2, le bras 2 est apte à se déplacer entre :
- une position rétractée (figure 1) facilitant son transport d'une salle d'intervention à une autre et/ou à l'intérieur d'une salle d'intervention, et
- une position déployée initiale (figure 2) préalablement au positionnement de son extrémité libre sur le tissu oculaire à traiter.

Le bras 2 est par exemple un TX260L commercialisé par la société STAUBLI.

Le déplacement du bras 2 est contrôlé par le dispositif de contrôle 5 qui :
- détermine à chaque instant la position courante dans l'espace de l'extrémité libre du bras,
- génère des consignes de déplacement afin d'ajuster la position courante de son extrémité libre en activant un ou plusieurs moteurs pour atteindre une position finale désirée - position dans laquelle l'organe d'immobilisation est centré et en contact avec le tissu oculaire,
- génère des consignes d'immobilisation du bras afin de maintenir le bras immobile en activant les freins.

Avantageusement, le bras peut comprendre des moyens de débrayage pour permettre son déplacement manuellement, par exemple en cas de défaillance ou de coupure de courant.

### 3. Système de découpe

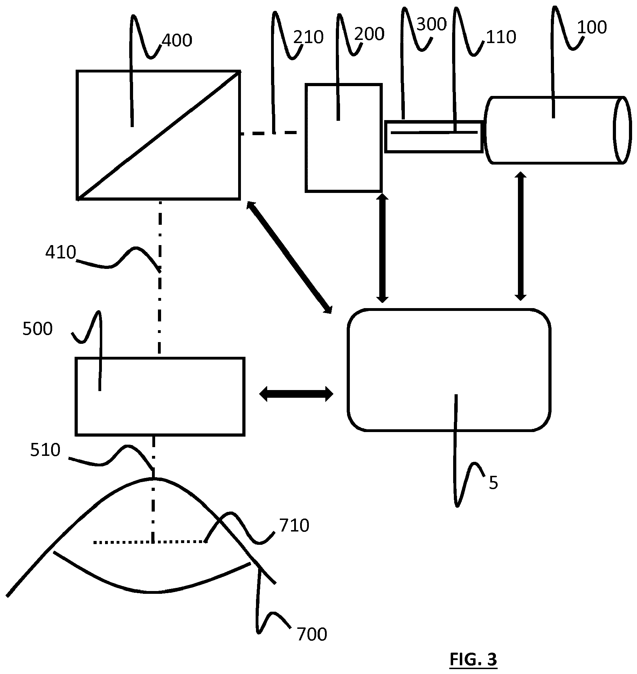

En référence à la figure 3, on a illustré un mode de réalisation d'un système de découpe utilisable avec l'appareil de thérapie selon l'invention. Le système de découpe comprend :
- un laser femtoseconde 100,
- un système de mise en forme 200 - tel qu'un modulateur spatial de lumière à cristaux liquides (ou *« SLM »*, acronyme de l'anglais *« Spatial Light Modulator »*) - positionné en aval du laser femtoseconde 100,
- un coupleur optique 300 entre le laser femtoseconde 100 et le système de mise en forme 200,
- un scanner optique de balayage 400 en aval du système de mise en forme 200,
- un système optique de focalisation 500 en aval du scanner optique de balayage 400.

Le dispositif de contrôle 5 permet de piloter le système de mise en forme 200, le scanner optique de balayage 400 et le système optique de focalisation 500.

Le laser femtoseconde 100 est apte à émettre un faisceau LASER initial sous la forme d'impulsions. Par *« laser femtoseconde »*, on entend une source lumineuse, apte à émettre un faisceau LASER sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

Le système de mise en forme 200 s'étend sur la trajectoire du faisceau LASER initial 110 issu du laser femtoseconde 100. Il permet de transformer le faisceau LASER initial 110 en un faisceau LASER modulé 210. Plus précisément, le système de mise en forme permet de moduler la phase du faisceau LASER 110 pour répartir l'énergie du faisceau LASER en une pluralité de points d'impact dans son plan focal, cette pluralité de points d'impact définissant un motif. En d'autres termes, le système de mise en forme 200 permet de moduler la répartition finale d'énergie du faisceau LASER dans le plan de focalisation 710 correspondant au plan de découpe du tissu 700. Il est adapté pour modifier le profil spatial du front d'onde du faisceau LASER primaire 110 issu du laser femtoseconde 100 pour distribuer l'énergie du faisceau LASER en différents points de focalisation dans le plan de focalisation 710. Le système de mise en forme 200 permet donc, à partir d'un faisceau LASER gaussien générant un unique point d'impact, et au moyen du masque de phase, de répartir son énergie par modulation de phase de sorte à générer simultanément plusieurs points d'impact dans son plan de focalisation à partir d'un unique faisceau LASER mis en forme par modulation de phase (un seul faisceau en amont et en aval du SLM).

Le coupleur optique 300 permet de transmettre le faisceau LASER 110 issu du laser femtoseconde 100 vers le système de mise en forme 200. Il comprend avantageusement une fibre optique, notamment une fibre à cristal photonique (ou *« PCF »*, sigle de l'expression anglo-saxonne *« Photonic-Crystal Fiber »*) à cœur creux. Une fibre à cristal photonique à cœur creux est une fibre optique qui guide la lumière essentiellement à l'intérieur d'une région creuse (le cœur de la fibre), de sorte que seule une partie mineure de la puissance optique se propage dans le matériau de fibre solide (typiquement un verre). Les attraits des fibres à cristal photonique à cœur creux sont principalement que le guidage primaire dans la région creuse minimise les effets non linéaires du faisceau LASER modulé et permet un seuil de dommage élevé. Avantageusement, la région creuse de la fibre à cristal photonique à cœur creux peut être mise sous vide pour limiter les pertes de propagation du faisceau LASER issu du laser femtoseconde 100. A cet effet, le coupleur optique 300 comprend des première et deuxième cellules de liaison montées de manière étanche à chaque extrémité de la fibre à cristal photonique à cœur creux. Ces cellules de liaison sont reliées à une pompe à vide P intégrée au caisson 1 pour mettre le cœur creux de la fibre optique sous vide par pompage au niveau des cellules de liaisons. Le fait de réaliser un pompage sous vide à chaque extrémité de la fibre optique 31 permet de faciliter la mise sous vide du cœur creux sur toute la longueur de la fibre optique 31.

Le scanner optique de balayage 400 permet d'orienter le faisceau LASER modulé 210 pour déplacer le motif le long d'un chemin de déplacement prédéfini par l'utilisateur dans un plan de focalisation 710.

Le système optique de focalisation 500 permet de déplacer le plan de focalisation 710 - correspondant au plan de découpe - du faisceau LASER dévié 410 issu du scanner optique de balayage 400.

Avantageusement, le système de mise en forme 200, le scanner optique de balayage 400 et le système optique de focalisation 500 peuvent être montés dans un compartiment fixé à l'extrémité 24 du bras, tandis que le laser femtoseconde peut être intégré au caisson 1, le coupleur optique 300 s'étendant entre le caisson 1 et le segment d'extrémité 24 pour propager le faisceau laser initial 110 entre le laser femtoseconde 100 et le système de mise en forme 200.

### 4. Capteur de force

Le capteur de force 3 permet de détecter des forces mécaniques générées en opposition à un mouvement du bras 2, ces forces traduisant la présence d'un obstacle et pouvant correspondre à l'obtention d'un contact entre l'extrémité du bras 2 et le tissu oculaire. Le capteur de force 3 peut être monté sur le segment d'extrémité 24 du bras 2.

Le capteur de force 3 est de type connu en soi par l'homme du métier. Il est apte à capter et mesurer des forces de compression et de traction appliquées le long de l'axe longitudinal du segment d'extrémité 24 du bras 2. Il comprend une (ou plusieurs) jauges de contrainte montée(s) sur le segment d'extrémité 24 du bras 2.

Chaque force mécanique mesurée par le capteur de force 3 est transmise au dispositif de contrôle 5.

Lorsque la valeur de la force mécanique mesurée est supérieure à une valeur seuil, le dispositif de contrôle effectue une ou plusieurs actions prédéterminées (génération d'une consigne d'immobilisation du bras, commande d'émission d'un stimulus visuel sur des moyens d'affichage et de saisie 12, et/ou d'un stimulus auditif sur un hautparleur intégré au caisson, etc.).

### 5. Système d'acquisition

Le système d'acquisition 4 permet d'acquérir des couples de mesure utilisés pour contrôler le déplacement du bras 2 par rapport au tissu oculaire à traiter.

Chaque couple de mesure comprend une (ou plusieurs) image(s) d'une zone située en regard de l'extrémité libre du bras 2.

A cet effet, le système d'acquisition 4 peut comprendre une unité d'acquisition d'image de type OCT (tomographie par cohérence optique) ou Scheimpflug (cartographie en lumière visible), ou UBM (Ultrasonic Bio Microscopy). Une telle unité d'acquisition d'image peut être montée sur le segment d'extrémité 24 du bras 2, par exemple en amont du scanner optique de balayage 400. Cette unité d'acquisition d'image est agencée de sorte à disposer d'un champ d'acquisition suffisamment large (par exemple observer un périmètre P correspondant à un carré de 50 cm de côté à une distance de 30 cm) afin de pouvoir identifier le tissu oculaire dans ce champ d'acquisition. Avantageusement, l'unité d'acquisition d'image peut être équipée de moyens d'éclairage (coaxiaux ou non) afin de faciliter la reconnaissance du tissu oculaire.

Chaque couple de mesure comprend également un (ou plusieurs) signal (signaux) représentatif(s) d'une distance entre l'extrémité libre du bras 2 et le tissu oculaire.

A cet effet, le système d'acquisition 4 peut comprendre une unité de télémétrie laser, ou une unité de télémétrie par ultrasons, soit ou une unité de télémétrie par analyse d'image, ou par tout autre dispositif équivalent connu de l'homme du métier capable d'acquérir un signal représentatif d'une distance entre l'extrémité libre du bras 2 et l'objet situé en regard de cette extrémité. Une telle unité de télémétrie peut également être montée sur le segment d'extrémité 24 du bras 2.

### 6. Dispositif de contrôle

Le dispositif de contrôle 5 permet de :
- traiter les couples de mesure issus du système d'acquisition ainsi que les forces mesurées par le capteur de force 3, et de
- piloter les différents éléments constituant l'appareil de thérapie (bras 2, système de découpe (notamment laser femtoseconde 100, système de mise en forme 200, scanner de balayage 400, système optique de focalisation 500, pompe à vide du coupleur optique 300, etc.), capteur de force 3, système d'acquisition 4, etc.).

Le dispositif de contrôle 5 est connectée à ces différents éléments par l'intermédiaire d'un (ou plusieurs) bus de communication permettant la transmission de signaux de commande, et la réception de données d'acquisition issues du capteur de force 3, du système d'acquisition 4, etc.

Le dispositif de contrôle 5 peut être composé d'une (ou plusieurs) station(s) de travail, et/ou d'un (ou plusieurs) ordinateur(s). Le dispositif de contrôle 5 comprend un processeur programmé pour permettre le pilotage des différents éléments de l'appareil de thérapie, et pour permettre le traitement des signaux acquis par le capteur de force 3 et le système d'acquisition 4.

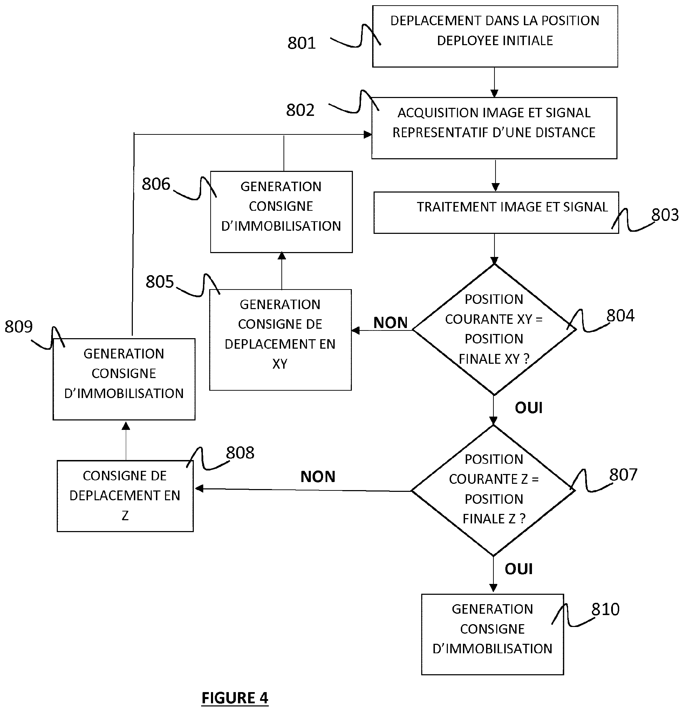

Le dispositif de contrôle 5 est programmé pour mettre en œuvre le procédé illustré à la figure 4. A cet effet, le dispositif de contrôle 5 comprend :
- des moyens de commande du système d'acquisition 4,
- des moyens de traitement de chaque couple de mesure acquis par le système d'acquisition 4, et
- des moyens d'asservissement pour générer des consignes de déplacement et d'immobilisation du bras 2.

Les moyens de commande permettent d'activer le système d'acquisition 4 pour acquérir une pluralité de couples de mesure successivement dans le temps. Plus précisément, après chaque émission d'une consigne d'immobilisation par les moyens d'asservissement, les moyens de commande émettent un signal d'activation du système d'acquisition pour l'acquisition d'un nouveau couple de mesure. Ce nouveau couple de mesure est traité par les moyens de traitement afin de mettre à jour l'écart entre la position courante de l'extrémité du bras et sa position finale désirée.

Les moyens de traitement sont aptes, à partir de chaque couple de mesure acquis, de détecter la position tridimensionnelle du tissu oculaire et la position tridimensionnelle de l'extrémité du bras.

La position tridimensionnelle de l'extrémité libre du bras est connue par construction.

La position tridimensionnelle du tissu oculaire est quant à elle obtenue par calcul à partir du couple de mesure issu du système d'acquisition 4. Par exemple dans l'image acquise par le système d'acquisition 4, les moyens de traitement sont capables d'identifier le tissu oculaire, sa position bidimensionnelle et son centre en reconnaissant une forme proche d'une morphologie typique d'un œil (trois cercles concentriques : un cercle blanc (la sclère), au centre duquel se trouve un cercle coloré (l'iris) au centre duquel se trouve un cercle noir (la pupille)). La troisième coordonnée nécessaire à l'estimation de la position tridimensionnelle du tissu oculaire est déduite du signal acquis par l'unité de télémétrie, ce signal étant représentatif de la distance entre l'extrémité libre du bras et le tissu oculaire.

Pour traiter chaque couple de mesure reçu du système d'acquisition 4, les moyens de traitement comprennent :
- des moyens d'estimation, à partir du couple de mesure courant, de la distance verticale selon l'axe Z entre l'extrémité du bras et le tissu oculaire,
- des moyens de calcul, à partir de l'image du couple de mesure courant, d'un écart horizontal entre :
   - la position horizontale courante de l'extrémité libre du bras dans le plan horizontal XY, et
   - la position horizontale finale désirée de l'extrémité libre du bras dans le plan horizontal XY.

Les moyens d'asservissement sont programmés pour mettre en œuvre une boucle d'asservissement dans le plan XY et une boucle d'asservissement selon la direction Z.

Avantageusement, le déplacement de l'extrémité libre du bras 2 selon les axes XY est décorrélé de son déplacement selon l'axe Z. En particulier, le dispositif de contrôle 5 est programmé pour :
- dans un premier temps déplacer l'extrémité libre du bras 2 dans un plan horizontal XY pour positionner ladite extrémité libre dans la position horizontale finale désirée, en interdisant tout déplacement de l'extrémité libre selon l'axe vertical Z (i.e. sans rapprocher l'extrémité libre du bras du tissu oculaire),
- dans un deuxième temps déplacer l'extrémité libre du bras 2 selon l'axe vertical Z pour la rapprocher du tissu oculaire jusqu'à obtention d'un contact, en interdisant tout déplacement de l'extrémité libre dans le plan horizontal XY.

Ceci permet d'éviter tout risque de blessure du patient (par exemple par frottement de l'extrémité libre du bras sur l'œil du patient si un déplacement dans le plan XY était commandé alors que l'extrémité est déjà en contact avec le tissu oculaire).

Les moyens d'asservissement du dispositif de contrôle 5 sont aptes à générer une pluralité de consignes de déplacement successives pour déplacer l'extrémité libre du bras de la position déployée initiale à la position finale désirée dans laquelle l'organe d'immobilisation est centré et en contact avec le tissu oculaire à traiter.

Plus précisément, si la distance entre la position courante de l'extrémité du bras 2 et la position finale désirée est supérieure à une valeur seuil, les moyens d'asservissement génèrent une pluralité de consignes de déplacement élémentaires successives pour amener l'extrémité du bras dans la position finale désirée.

Entre chaque émission d'une consigne de déplacement élémentaires, les moyens d'asservissement génèrent une consigne d'immobilisation, et les moyens de commande émettent un signal d'activation du système d'acquisition 4 afin d'acquérir un nouveau couple de mesure. Ceci permet de vérifier tout au long du déplacement du bras 2 que son extrémité se rapproche de la position finale désirée, et de tenir compte d'un éventuel mouvement inopiné de la tête du patient (auquel cas la position finale désirée est mise à jour).

### 7. Principe de fonctionnement

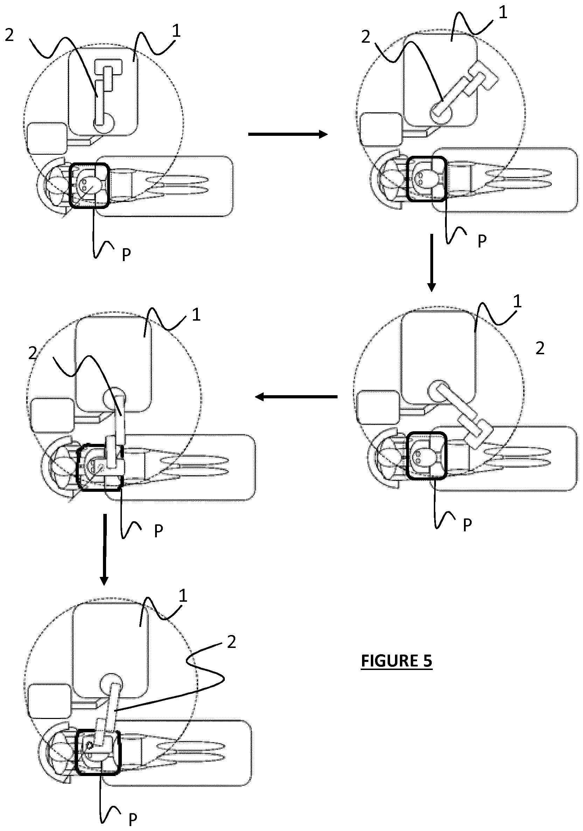

On va maintenant décrire plus en détails le principe de fonctionnement de l'équipement de thérapie en référence aux figures 4 et 5.

### 7.1. Préalable à l'utilisation de l'appareil de thérapie

En condition préalable au bon fonctionnement de l'appareil de thérapie décrit précédemment, il convient de préciser qu'il sera nécessaire dans chaque endroit où cet appareil est utilisé, de définir la position de l'équipement chirurgical dans la salle avec un marquage au sol, position qui sera définie en fonction :
- des préférences du chirurgien (position droite ou gauche, plutôt orientée devant, latéral ou plutôt derrière, plutôt proche ou éloigné)
- de la position habituelle finale du lit sur lequel est allongé le patient
- de la forme du lit, ses dimensions, sa hauteur
- du respect d'une contrainte de distance, en faisant en sorte que la position finale de la tête du patient, se trouve dans un périmètre centré autour du point de fixation du bras robotisé sur l'équipement chirurgical, symbolisant son rayon d'action, ou distance au-delà de laquelle le bras ne pourra plus atteindre sa cible.

Une fois le marquage au sol défini, l'appareil de thérapie sera positionné sur le même emplacement à chaque utilisation. De cette manière, la position relative de chaque patient par rapport à la machine et notamment de sa tête et de ses yeux, est connue avec une marge d'erreur acceptable, pouvant aller jusqu'à 20 centimètres.

Ainsi, il est possible par paramétrage accessible via l'interface homme machine de l'appareil, de définir les coordonnées d'un périmètre P correspondant à un carré de 50cm de côté, dans lequel se trouvera la tête de chaque patient s'apprêtant à recevoir une thérapie oculaire avec le système objet de la présente invention (périmètre P de présence certaine de la cible). Une fois les coordonnées de ce périmètre P mémorisées, à chaque utilisation, le dispositif de contrôle 5 commande le positionnement de l'extrémité du bras (par défaut et avant les itérations nécessaires à l'obtention d'un centrage parfait) au milieu du périmètre P. Cette position correspond à la position déployée initiale.

Le centrage et la mise en contact de l'organe d'immobilisation sur le tissu oculaire sont réalisés comme suit.

### 7.2. Positionnement automatique de l'extrémité libre du bras sur le tissu oculaire

### 7.2.1. Déploiement du bras

Une fois le patient installé et l'appareil de thérapie en place, le dispositif de contrôle 5 commande le déploiement du bras 2 (étape 801).

Le bras 2 se déplace automatiquement (comme illustré sur les quatre premières étapes de la figure 5) de manière à positionner l'extrémité libre du bras 2 au centre du périmètre P (position déployée initiale).

Une fois le centre du périmètre P atteint, les itérations de la boucle d'asservissement XY sont lancées.

### 7.2.2. Boucle d'asservissement en XY

La boucle d'asservissement en XY est une fonction programmée qui, à chaque itération :
- reçoit une image,
- l'analyse,
- identifie les coordonnées XY de la position horizontale finale désirée,
- détermine les coordonnées X', Y' de la position horizontale courante de l'extrémité libre du bras 2, et
- calcule l'écart entre la position horizontale courante et position horizontale finale désirée,
- calcule la trajectoire restante entre position horizontale courante et position horizontale finale désirée,
- envoie au bras 2 une (ou plusieurs) consigne(s) de déplacement pour mettre le bras 2 en mouvement selon la trajectoire déterminée par calcul, et ceci jusqu'au moment où l'analyse de l'image reçue détermine que la position horizontale finale désirée est atteinte (X=X' et Y=Y') : l'extrémité libre du bras 2 est alors alignée avec un axe vertical passant par le centre du tissu oculaire.

Plus précisément, les moyens de commande du dispositif de contrôle 5 émettent un signal d'activation du système d'acquisition 4. Le système d'acquisition 4 acquiert une image et un signal représentatif de la distance entre l'extrémité du bras et le tissu oculaire.

Les moyens de traitement reçoivent le couple de mesure acquis par le système d'acquisition 4 et le traitent (étape 803).

En particulier, les moyens de traitement :
- détectent dans l'image acquise le tissu oculaire,
- déterminent la position du centre du tissu oculaire,
- définissent cette position du centre du tissu oculaire comme correspondant à la position horizontale finale désirée,
- estiment la position horizontale courante de l'extrémité libre du bras, et
- comparent (étape 804) la position horizontale courante à la position horizontale finale désirée (par exemple en calculant la distance entre la position horizontale courante et la position horizontale finale désirée).

Le résultat de cette comparaison est transmis aux moyens d'asservissement qui :
- commandent la mise en œuvre de la boucle d'asservissement en Z si la position horizontale courante coïncide avec la position horizontale finale désirée,
- génère une consigne de déplacement horizontal du bras 2 sinon (étape 805).

Une fois le bras 2 déplacé conformément à la consigne de déplacement, les moyens d'asservissement génèrent une consigne d'immobilisation (étape 806) du bras 2 et les étapes précédentes (d'activation du système d'acquisition 4, de traitement du couple de mesure, etc.) sont réitérées jusqu'à ce que la position horizontale finale désirée en XY soit atteinte par l'extrémité libre du bras 2.

### 7.2.3. Boucle d'asservissement en Z

Une fois l'extrémité libre du bras 2 alignée en XY avec la position horizontale finale désirée, la boucle d'asservissement en Z peut être mise en œuvre.

La boucle d'asservissement en Z est une fonction programmée qui, à chaque itération :
- reçoit une donnée d'altitude courante de l'extrémité du bras 2 par rapport au tissu oculaire (position Z' courante - position Z désirée),
- calcule l'écart entre la position verticale courante de l'extrémité du bras et la position verticale finale désirée,
- calcule la trajectoire restante entre position verticale courante et position verticale finale désirée,
- envoie au bras 2 une (ou plusieurs) consigne(s) de déplacement pour mettre le bras 2 en mouvement sur l'axe Z, sans changer de position XY, selon la trajectoire déterminée par calcul, et ceci jusqu'au moment où le capteur de force 3 détecte un contact traduisant le fait que la position verticale finale désirée est atteinte (Z'=Z) : l'extrémité libre du bras 2 est alors en contact avec le tissu oculaire.

Plus précisément, les moyens de traitement du dispositif de contrôle 5 traitent le signal représentatif d'une distance verticale selon l'axe Z (étape 803), et comparent (étape 807) la position verticale courante à la position verticale finale désirée.

Le résultat de cette comparaison est transmis aux moyens d'asservissement qui reçoivent également un signal mesuré par le capteur de force 3. Les moyens d'asservissement :
- génèrent une consigne d'immobilisation du bras 2 si la position verticale courante coïncide avec la position verticale finale désirée (étape 810),
- génèrent une consigne de déplacement vertical du bras 2 sinon (étape 808).

Une fois le bras 2 déplacé conformément à la consigne de déplacement vertical, les moyens d'asservissement génèrent une consigne d'immobilisation (étape 809) du bras 2 et les étapes précédentes sont réitérées, y compris les étapes de la boucle d'asservissement en XY, afin de vérifier que la position horizontale courante correspond toujours à la position horizontale finale désirée.

Ceci permet de tenir compte d'éventuels mouvements du patient au cours de la procédure de positionnement du bras 2.

Le dispositif de contrôle 5 permet de positionner l'extrémité libre du bras de manière précise et centrée. Cette extrémité libre porte les différents composants de travail permettant le traitement du tissu oculaire.

De manière utile, et rassurante pour le praticien, l'enchaînement des différentes étapes illustrées à la figure 4 pourra être contrôlé grâce à une pédale de commande, et/ou grâce à une commande vocale et/ou grâce à une interface homme machine tactile ou non.

### 8. Conclusions

L'invention décrite précédemment permet en quelques secondes de positionner automatiquement sur l'œil d'un patient, un organe d'immobilisation du globe oculaire, sans intervention humaine, de manière rapide, précise et répétable. Ses performances sont indépendantes de l'environnement, afin de gagner en précision, de rendre le geste reproductible quel que soit le patient ou l'opérateur, et de réaliser une économie de temps en soulageant l'opérateur d'une tâche à faible valeur ajoutée.

L'invention permet en outre d'apporter plus de sécurité et donc de réduire le risque encouru par le patient au moment de l'intervention.

Le lecteur aura compris que de nombreuses modifications peuvent être apportées à l'invention décrite précédemment sans sortir matériellement des nouveaux enseignements et des avantages décrits ici. Par exemple dans la description qui précède, l'organe d'immobilisation était monté sur l'extrémité libre du bras robotisé. En variante, l'organe d'immobilisation peut être dissocié du bras robotisé. Dans ce cas, l'organe d'immobilisation est positionné sur l'œil du patient préalablement au déplacement du bras robotisé, et la position finale désirée correspond à la mise en contact de l'extrémité libre du bras robotisé avec une face de l'organe d'immobilisation opposée à la surface de l'organe d'immobilisation en contact avec l'œil. Par conséquent, toutes les modifications de ce type sont destinées à être incorporées à l'intérieur de la portée des revendications jointes.

## Revendications

1. Dispositif de contrôle (5) du déplacement d'un appareil de thérapie oculaire du type comprenant :
- un bras support (2), l'extrémité libre du bras (2) étant destinée à venir au droit d'un tissu oculaire humain ou animal, ledit bras (2) étant articulé pour permettre le déplacement de l'extrémité libre du bras (2) selon trois axes X, Y et Z orthogonaux deux à deux :
• l'axe X, définissant une direction longitudinale, horizontale,
• l'axe Y, définissant une direction transversale, horizontale, qui avec l'axe X définit un plan XY horizontal,
• l'axe Z, définissant une direction verticale, perpendiculaire au plan XY horizontal,
- un système d'acquisition (4) monté sur le bras (2) pour l'acquisition d'un couple de mesure comportant :
• une image du tissu oculaire, et
• un signal représentatif d'une distance verticale selon l'axe Z entre l'extrémité du bras (2) et le tissu oculaire, le dispositif de contrôle (5) comprend :
- des moyens de commande du système d'acquisition (4) pour l'acquisition d'une pluralité de couples de mesure successivement dans le temps,
- des moyens de traitement de chaque couple de mesure, lesdits moyens de traitement incluant :
• des moyens d'estimation, à partir du couple de mesure courant, de la distance verticale selon l'axe Z entre l'extrémité du bras et le tissu oculaire,
• des moyens de calcul, à partir de l'image du couple de mesure courant, d'un écart horizontal entre :
▪ une position horizontale courante de l'extrémité libre du bras dans le plan horizontal XY, et
▪ une position horizontale finale désirée de l'extrémité libre du bras dans le plan horizontal XY,
**caractérisé en ce que** le dispositif comprend :
- des moyens d'asservissement pour :
• si l'écart horizontal calculé est supérieur à une première valeur seuil, générer une consigne de déplacement horizontal du bras (2) dans le plan horizontal XY pour réduire l'écart entre la position horizontale courante et la position horizontale finale désirée,
• si l'écart horizontal calculé est inférieur à la première valeur seuil et si la distance verticale estimée est supérieure à une deuxième valeur seuil, générer une consigne de déplacement vertical du bras (2) selon une direction verticale pour réduire la distance entre l'extrémité libre du bras et le tissu oculaire,
• si l'écart horizontal calculé est inférieur à la première valeur seuil et si la distance verticale mesurée est inférieure à la deuxième valeur seuil, générer une consigne d'immobilisation du bras.

2. Dispositif de contrôle selon la revendication 1, ***dans** lequel* les moyens de calcul comprennent :
- des moyens de détection, à partir de l'image du couple de mesure courant, de la position horizontale d'au moins un point d'intérêt du tissu oculaire,
- des moyens d'évaluation, à partir de la position horizontale détectée du point d'intérêt, d'un écart horizontal entre :
• la position horizontale courante de l'extrémité libre du bras dans le plan horizontal XY, et
• la position horizontale finale désirée de l'extrémité libre du bras dans le plan horizontal XY.

3. Dispositif de contrôle selon la revendication 2, ***dans lequel*** les moyens de détection sont aptes à identifier le tissu oculaire dans l'image acquise, par la mise en œuvre d'un algorithme de reconnaissance de forme pour détecter trois cercles concentriques dans l'image.

4. Dispositif de contrôle selon l'une quelconque des revendications 1 à 3, ***dans lequel*** l'appareil de thérapie comprend en outre un capteur de force (3) monté sur l'extrémité libre du bras pour mesurer une force mécanique appliquée sur l'extrémité libre du bras :
- les moyens de traitement comprenant des moyens de comparaison de ladite force mécanique mesurée à une troisième valeur seuil pour déterminer si l'extrémité libre du bras est en contact avec un élément faisant obstacle à un déplacement vertical du bras selon l'axe Z,
- les moyens d'asservissement étant programmés pour générer une consigne d'immobilisation du bras si la force mécanique mesurée est supérieure à la troisième valeur seuil.

5. Dispositif de contrôle selon l'une quelconque des revendications 1 à 4, ***dans lequel*** le système d'acquisition comprend, pour l'acquisition d'un signal représentatif d'une distance verticale selon l'axe Z :
- des moyens d'acquisition par télémétrie laser, et/ou
- des moyens d'acquisition par ultrasons
- des moyens d'acquisition par traitement d'image.

6. Dispositif de contrôle selon l'une quelconque des revendications précédentes, ***dans lequel*** les moyens d'asservissement sont programmés pour générer des consignes de déplacement élémentaires pour permettre le déplacement du bras entre sa position courante et une position finale désirée, lesdits moyens d'asservissement générant une consigne d'immobilisation consécutive à chaque consigne de déplacement élémentaire.

7. Procédé de contrôle du déplacement d'un appareil de thérapie oculaire du type comprenant :
- un bras support (2), l'extrémité libre du bras (2) étant destinée à venir au droit d'un tissu oculaire humain ou animal, ledit bras (2) étant articulé pour permettre le déplacement de l'extrémité libre du bras (2) selon trois axes X, Y et Z orthogonaux deux à deux :
• l'axe X, définissant une direction longitudinale, horizontale,
• l'axe Y, définissant une direction transversale, horizontale, qui avec l'axe X définit un plan XY horizontal,
• l'axe Z, définissant une direction verticale, perpendiculaire au plan XY horizontal,
- un système d'acquisition (4) monté sur le bras (2) pour l'acquisition d'un couple de mesure comportant :
• une image du tissu oculaire, et
• un signal représentatif d'une distance verticale selon l'axe Z entre l'extrémité du bras et le tissu oculaire,
***caractérisé en ce que*** le procédé de contrôle comprend les phases suivantes :
- acquérir (802) une pluralité de couples de mesure successivement dans le temps grâce au système d'acquisition,
- traiter (803) chaque couple de mesure, la phase de traitement comprenant les étapes consistant en :
• l'estimation à partir du couple de mesure courant, de la distance verticale selon l'axe Z entre l'extrémité du bras et le tissu oculaire,
• le calcul à partir de l'image du couple de mesure courant, d'un écart horizontal entre :
▪ une position horizontale courante de l'extrémité libre du bras dans le plan horizontal XY, et
▪ une position horizontale finale désirée de l'extrémité libre du bras dans le plan horizontal XY,
- asservir le déplacement du bras (2) en :
• si l'écart horizontal calculé est supérieur à une première valeur seuil, générant (805) une consigne de déplacement horizontal du bras (2) dans le plan horizontal XY pour réduire l'écart entre la position horizontale courante et la position horizontale finale désirée,
• si l'écart horizontal calculé est inférieur à la première valeur seuil et si la distance verticale estimée est supérieure à une deuxième valeur seuil, générant (808) une consigne de déplacement vertical du bras (2) selon une direction verticale pour réduire la distance entre l'extrémité libre du bras et le tissu oculaire,
• si l'écart horizontal calculé est inférieur à la première valeur seuil et si la distance verticale mesurée est inférieure à la deuxième valeur seuil, générant (810) une consigne d'immobilisation du bras.

8. Procédé de contrôle selon la revendication 7, ***dans lequel*** l'étape de calcul comporte les sous-étapes suivantes :
- la détection, à partir de l'image du couple de mesure courant, de la position horizontale d'au moins un point d'intérêt du tissu oculaire,
- l'évaluation, à partir de la position horizontale détectée du point d'intérêt, d'un écart horizontal entre :
• la position horizontale courante de l'extrémité libre du bras dans le plan horizontal XY, et
• la position horizontale finale désirée de l'extrémité libre du bras dans le plan horizontal XY.

9. Procédé de contrôle selon la revendication 8, ***dans lequel*** la sous-étape de détection consiste à identifier le tissu oculaire dans l'image acquise, par la mise en œuvre d'un algorithme de reconnaissance de forme pour détecter trois cercles concentriques dans l'image.

10. Procédé de contrôle selon l'une quelconque des revendications 7 à 9, ***dans lequel*** l'appareil de thérapie comprend en outre un capteur de force (3) monté sur l'extrémité libre du bras (2) pour mesurer une force mécanique appliquée sur l'extrémité libre du bras (2) :
- la phase de traitement comprenant une étape de comparaison de ladite force mécanique mesurée à une troisième valeur seuil pour déterminer si l'extrémité libre du bras est en contact avec un élément faisant obstacle à un déplacement vertical du bras selon l'axe Z,
- l'étape d'asservissement incluant la génération d'une consigne d'immobilisation du bras si la force mécanique mesurée est supérieure à la troisième valeur seuil.

11. Procédé de contrôle selon l'une quelconque des revendications 7 à 10, ***dans lequel*** la phase consistant à acquérir comprend :
- l'acquisition par télémétrie laser d'un signal représentatif d'une distance verticale selon l'axe Z, et/ou
- l'acquisition par ultrasons d'un signal représentatif d'une distance verticale selon l'axe Z, et/ou,
- l'extraction d'une image acquise d'un signal représentatif d'une distance verticale selon l'axe Z.

12. Procédé de contrôle selon l'une quelconque des revendications 7 à 11, ***dans lequel*** l'étape d'asservissement comporte :
- générer une consigne de déplacement élémentaire pour permettre le déplacement du bras entre sa position courante et une position finale désirée,
- générer une consigne d'immobilisation consécutive à chaque consigne de déplacement élémentaire,
- répéter les sous-étapes précédentes jusqu'à ce que l'écart horizontal calculé soit inférieur à la première valeur seuil et que la distance verticale mesurée soit inférieure à la deuxième valeur seuil.

## Patentansprüche

1. Vorrichtung zur Steuerung (5) der Bewegung einer Augentherapievorrichtung des Typs, der umfasst:
- einen Stützarm (2), wobei das freie Ende des Arms (2) bestimmt ist, gegenüber einem Augengewebe eines Menschen oder eines Tieres zu kommen, wobei der Arm (2) gelenkig ist, um die Bewegung des freien Endes des Arms (2) in drei Achsen X, Y und Z zu gestatten, die paarweise orthogonal sind:
o in der eine Längsrichtung definierenden horizontalen Achse X,
o in der eine Querrichtung definierenden horizontalen Achse Y, die mit der Achse X eine horizontale Ebene XY definiert,
o in der eine vertikale Richtung definierenden Achse Z, die zu der horizontalen Ebene XY senkrecht ist,
- ein Erfassungssystem (4), das auf dem Arm (2) für die Erfassung eines Messpaares angebracht ist, das aufweist:
o ein Bild des Augengewebes, und
o ein Signal, das für einen vertikalen Abstand gemäß der Achse Z zwischen dem Ende des Arms (2) und dem Augengewebe repräsentativ ist,
wobei die Steuerungsvorrichtung (5) umfasst:
- Steuermittel des Erfassungssystems (4) für die zeitlich aufeinanderfolgende Erfassung einer Vielzahl von Messpaaren,
- Verarbeitungsmittel jedes Messpaares, wobei die Verarbeitungsmittel einschließen:
o Schätzmittel, ausgehend vom laufenden Messpaar, des vertikalen Abstands gemäß der Achse Z zwischen dem Ende des Arms und dem Augengewebe,
o Mittel zum Berechnen, ausgehend vom Bild des laufenden Messpaares, einer horizontalen Abweichung zwischen:
▪ einer laufenden horizontalen Position des freien Endes des Arms in der horizontalen Ebene XY, und
▪ einer gewünschten endgültigen horizontalen Position des freien Endes des Arms in der horizontalen Ebene XY,
**dadurch gekennzeichnet, dass** die Vorrichtung umfasst:
- Regelungsmittel, um:
∘ wenn die berechnete horizontale Abweichung größer als ein erster Grenzwert ist, einen Sollwert der horizontalen Bewegung des Arms (2) in der horizontalen Ebene XY zu erzeugen, um die Abweichung zwischen der laufenden horizontalen Position und der gewünschten endgültigen horizontalen Position zu reduzieren,
∘ wenn die berechnete horizontale Abweichung kleiner als der erste Grenzwert ist und wenn der geschätzte vertikale Abstand größer als ein zweiter Grenzwert ist, einen Sollwert der vertikalen Bewegung des Arms (2) gemäß einer vertikalen Richtung zu erzeugen, um den Abstand zwischen dem freien Ende des Arms und dem Augengewebe zu reduzieren,
∘ wenn die berechnete horizontale Abweichung kleiner als der erste Grenzwert ist und wenn der gemessene vertikale Abstand kleiner als der zweite Grenzwert ist, einen Sollwert für die Arretierung des Arms zu erzeugen.

2. Steuerungsvorrichtung nach Anspruch 1, wobei die Rechenmittel umfassen:
- Mittel zur Detektion, ausgehend von dem Bild des laufenden Messpaares, der horizontalen Position von mindestens einem interessierenden Punkt des Augengewebes,
- Mittel zur Evaluierung, ausgehend von der detektierten horizontalen Position des interessierenden Punkts, einer horizontalen Abweichung zwischen:
∘ der laufenden horizontalen Position des freien Endes des Arms in der horizontalen Ebene XY, und
∘ der gewünschten endgültigen horizontalen Position des freien Endes des Arms in der horizontalen Ebene XY.

3. Steuerungsvorrichtung nach Anspruch 2, wobei die Detektionsmittel imstande sind, das Augengewebe im erfassten Bild durch Einsatz eines Formerkennungsalgorithmus zur Detektion von drei konzentrischen Kreisen im Bild zu identifizieren.

4. Steuerungsvorrichtung nach einem der Ansprüche 1 bis 3, wobei das Therapiegerät ferner einen Kraftsensor (3) umfasst, der auf dem freien Ende des Arms angebracht ist, um eine auf das freie Ende des Arms angewendete mechanische Kraft zu messen:
- wobei die Verarbeitungsmittel Mittel zum Vergleichen der gemessenen mechanischen Kraft mit einem dritten Grenzwert umfassen, um zu ermitteln, ob das freie Ende des Arms mit einem Element im Kontakt ist, das eine vertikale Bewegung des Arms gemäß der Achse Z behindert,
- wobei die Regelungsmittel programmiert sind, um einen Arretierungs-Sollwert des Arms zu erzeugen, wenn die gemessene mechanische Kraft größer als der dritte Grenzwert ist.

5. Steuerungsvorrichtung nach einem der Ansprüche 1 bis 4, wobei das Erfassungssystem für die Erfassung eines Signals, das für einen vertikalen Abstand gemäß der Achse Z repräsentativ ist, umfasst:
- Erfassungsmittel durch Lasertelemetrie, und/oder
- Erfassungsmittel durch Ultraschall,
- Erfassungsmittel durch Bildbearbeitung.

6. Steuerungsvorrichtung nach einem der vorangehenden Ansprüche, wobei die Regelungsmittel programmiert sind, um elementare Bewegungs-Sollwerte zu erzeugen, um die Bewegung des Arms zwischen seiner laufenden Position und einer gewünschten endgültigen Position zu erlauben, wobei die Regelungsmittel infolge jedes elementaren Bewegungs-Sollwerts einen Arretierungs-Sollwert erzeugen.

7. Verfahren zur Steuerung der Bewegung einer Augentherapievorrichtung des Typs, der umfasst:
- einen Stützarm (2), wobei das freie Ende des Arms (2) bestimmt ist, gegenüber einem Augengewebe eines Menschen oder eines Tieres zu kommen, wobei der Arm (2) gelenkig ist, um die Bewegung des freien Endes des Arms (2) in drei Achsen X, Y und Z zu gestatten, die paarweise orthogonal sind:
∘ in der eine Längsrichtung definierenden horizontalen Achse X,
∘ in der eine Querrichtung definierenden horizontalen Achse Y, die mit der Achse X eine horizontale Ebene XY definiert,
∘ in der eine vertikale Richtung definierenden Achse Z, die zu der horizontalen Ebene XY senkrecht ist,
- ein Erfassungssystem (4), das auf dem Arm (2) für die Erfassung eines Messpaares angebracht ist, das aufweist:
∘ ein Bild des Augengewebes, und
∘ ein Signal, das für einen vertikalen Abstand gemäß der Achse Z zwischen dem Ende des Arms und dem Augengewebe repräsentativ ist,
**dadurch gekennzeichnet, dass** das Steuerungsverfahren die folgenden Phasen umfasst:
- zeitlich aufeinanderfolgendes Erfassen (802) einer Vielzahl von Messpaaren dank des Erfassungssystems,
- Verarbeiten (803) jedes Messpaares, wobei die Verarbeitungsphase Schritte umfasst, die bestehen im:
∘ Schätzen, ausgehend vom laufenden Messpaar, des vertikalen Abstands gemäß der Achse Z zwischen dem Ende des Arms und dem Augengewebe,
∘ Berechnen, ausgehend vom Bild des laufenden Messpaares, einer horizontalen Abweichung zwischen:
▪ einer laufenden horizontalen Position des freien Endes des Arms in der horizontalen Ebene XY, und
▪ einer gewünschten endgültigen horizontalen Position des freien Endes des Arms in der horizontalen Ebene XY,
- Regeln der Bewegung des Arms (2) durch:
∘ wenn die berechnete horizontale Abweichung größer als ein erster Grenzwert ist, Erzeugen (805) eines Sollwerts der horizontalen Bewegung des Arms (2) in der horizontalen Ebene XY, um die Abweichung zwischen der laufenden horizontalen Position und der gewünschten endgültigen horizontalen Position zu reduzieren,
∘ wenn die berechnete horizontale Abweichung kleiner als der erste Grenzwert ist und wenn der geschätzte vertikale Abstand größer als ein zweiter Grenzwert ist, Erzeugen (808) eines Sollwerts der vertikalen Bewegung des Arms (2) gemäß einer vertikalen Richtung, um den Abstand zwischen dem freien Ende des Arms und dem Augengewebe zu reduzieren,
∘ wenn die berechnete horizontale Abweichung kleiner als der erste Grenzwert ist und wenn der gemessene vertikale Abstand kleiner als der zweite Grenzwert ist, Erzeugen (810) eines Sollwerts für die Arretierung des Arms.

8. Steuerungsverfahren nach Anspruch 7, wobei der Berechnungsschritt die folgenden Unterschritte aufweist:
- Detektieren, ausgehend von dem Bild des laufenden Messpaares, der horizontalen Position von mindestens einem interessierenden Punkt des Augengewebes,
- Evaluieren, ausgehend von der detektierten horizontalen Position des interessierenden Punkts, einer horizontalen Abweichung zwischen:
o der laufenden horizontalen Position des freien Endes des Arms in der horizontalen Ebene XY, und
∘ der gewünschten endgültigen horizontalen Position des freien Endes des Arms in der horizontalen Ebene XY.

9. Steuerungsverfahren nach Anspruch 8, wobei der Unterschritt des Detektierens darin besteht, das Augengewebe im erfassten Bild durch Einsatz eines Formerkennungsalgorithmus zur Detektion von drei konzentrischen Kreisen im Bild zu identifizieren.

10. Steuerungsverfahren nach einem der Ansprüche 7 bis 9, wobei das Therapiegerät ferner einen Kraftsensor (3) umfasst, der auf dem freien Ende des Arms (2) angebracht ist, um eine auf das freie Ende des Arms (2) angewendete mechanische Kraft zu messen:
- wobei die Verarbeitungsphase einen Schritt des Vergleichens der gemessenen mechanischen Kraft mit einem dritten Grenzwert umfasst, um zu ermitteln, ob das freie Ende des Arms mit einem Element im Kontakt ist, das eine vertikale Bewegung des Arms gemäß der Achse Z behindert,
- wobei der Regelungsschritt das Erzeugen eines Arretierungs-Sollwerts des Arms einschließt, wenn die gemessene mechanische Kraft größer als der dritte Grenzwert ist.

11. Steuerungsverfahren nach einem der Ansprüche 7 bis 10, wobei die Phase, die darin besteht zu erfassen, umfasst:
- das Erfassen eines Signals durch Lasertelemetrie, das für einen vertikalen Abstand gemäß der Achse Z repräsentativ ist, und/oder
- das Erfassen eines Signals durch Ultraschall, das für einen vertikalen Abstand gemäß der Achse Z repräsentativ ist, und/oder
- das Extrahieren eines erfassten Bildes eines Signals, das für einen vertikalen Abstand gemäß der Achse Z repräsentativ ist.

12. Steuerungsverfahren nach einem der Ansprüche 7 bis 11, wobei der Regelungsschritt aufweist:
- Erzeugen eines elementaren Bewegungs-Sollwerts, um die Bewegung des Arms zwischen seiner laufenden Position und einer gewünschten endgültigen Position zu erlauben,
- Erzeugen eines Arretierungs-Sollwerts infolge jedes elementaren Bewegungs-Sollwerts,
- Wiederholen der vorangehenden Unterschritte, bis die berechnete horizontale Abweichung kleiner als der erste Grenzwert ist und der gemessene vertikale Abstand kleiner als der zweite Grenzwert ist.

## Claims

1. A monitoring device (5) for monitoring the movement of an ocular therapy apparatus of the type comprising:
- a support arm (2), the free end of the arm (2) being configured to come in line with a human or animal ocular tissue, said arm (2) being articulated to allow the movement of the free end of the arm (2) along three axes X, Y and Z which are orthogonal two by two:
∘ the X axis defining a longitudinal direction extending horizontally,
∘ the Y axis defining a transverse direction extending horizontally, the X axis and the Y axis defining a horizontal plane XY,
∘ the Z axis defining a vertical direction, perpendicular to the horizontal plane XY,
- an acquisition system (4) mounted on the arm (2) for the acquisition of a measurement pair including:
∘ an image of the ocular tissue, and
∘ a signal representative of a vertical distance along the Z axis between the end of the arm (2) and the ocular tissue,
- control means which control the acquisition system (4) to acquire a plurality of measurement pairs successively over time,
- processing means which process each measurement pair, said processing means including:
∘ estimation means which estimate, from the current measurement pair, the vertical distance along the Z axis between the end of the arm and the ocular tissue,
∘ calculation means which calculate, from the image of the current measurement pair, a horizontal deviation between:
▪ a current horizontal position of the free end of the arm in the horizontal plane XY, and
▪ a desired final horizontal position of the free end of the arm in the horizontal plane XY,
**characterized in that** the device comprises:
- servo-control means to:
∘ generate, if the calculated horizontal deviation is greater than a first threshold value, an instruction to horizontally move the arm (2) in the horizontal plane XY in order to reduce the deviation between the current horizontal position and the desired final horizontal position,
∘ generate, if the calculated horizontal deviation is less than the first threshold value and if the estimated vertical distance is greater than a second threshold value, an instruction to vertically move the arm (2) along a vertical direction in order to reduce the distance between the free end of the arm and the ocular tissue,
o generate, if the calculated horizontal deviation is less than the first threshold value and if the measured vertical distance is less than the second threshold value, an instruction to immobilize the arm.

2. The monitoring device according to claim 1, *wherein* the calculation means comprise:
∘ calculation means which detect, from the image of the current measurement pair, the horizontal position of at least one point of interest of the ocular tissue,
∘ evaluation means which evaluate, from the detected horizontal position of the point of interest, a horizontal deviation between:
▪ the current horizontal position of the free end of the arm in the horizontal plane XY, and
▪ the desired final horizontal position of the free end of the arm in the horizontal plane XY.

3. The monitoring device according to claim 2, *wherein* the detection means are able to identify the ocular tissue in the acquired image, by the implementation of a shape recognition algorithm in order to detect three concentric circles in the image.

4. The monitoring device according to any one of claims 1 to 3, *wherein* the ocular therapy apparatus further comprises a force sensor (3) mounted on the free end of the arm to measure a mechanical force applied to the free end of the arm, wherein:
- the processing means comprising comparison means which compare said measured mechanical force with a third threshold value to determine whether the free end of the arm is in contact with an element that obstructs a vertical movement of the arm along the Z axis,
- the servo-control means being programmed to generate an instruction to immobilize the arm if the measured mechanical force is greater than the third threshold value.

5. The monitoring device according to any one of claims 1 to 4, *wherein* the acquisition system comprises, for the acquisition of a signal representative of a vertical distance along the Z axis:
∘ means for acquiring by laser ranging, and/or
∘ means for acquiring by ultrasounds,
∘ means for acquiring by image processing.

6. The monitoring device according to any one of the preceding claims, *wherein* the servo-control means are programmed to generate elementary movement instructions to allow the movement of the arm between its current position and a desired final position, said servo-control means generating an immobilization instruction subsequent to each elementary movement instruction.

7. A monitoring method for monitoring the movement of an ocular therapy apparatus of the type comprising:
- a support arm (2), the free end of the arm (2) bing intended to come in line with a human or animal ocular tissue, said arm (2) being articulated to allow the movement of the free end of the arm (2) along three axes X, Y and Z which are orthogonal two by two:
∘ the X axis defining a longitudinal direction extending horizontally,
∘ the Y axis defining a transverse direction extending horizontally, the X axis and the Y axis defining a horizontal plane XY,
∘ the Z axis defining a vertical direction, perpendicular to the horizontal plane XY,
- an acquisition system (4) mounted on the arm (2) for the acquisition of a measurement pair including:
∘ an image of the ocular tissue, and
∘ a signal representative of a vertical distance along the Z axis between the end of the arm and the ocular tissue,
***characterized in that*** the monitoring method comprises the following phases:
- acquiring (802) a plurality of measurement pairs successively over time via the acquisition system,
- processing (803) each measurement pair, the processing phase comprising the steps consisting of:
∘ estimating, from the current measurement pair, the vertical distance along the Z axis between the end of the arm and the ocular tissue,
∘ calculating, from the image of the current measurement pair, a horizontal deviation between:
▪ a current horizontal position of the free end of the arm in the horizontal plane XY, and
▪ a desired final horizontal position of the free end of the arm in the horizontal plane XY,
- servo-controlling the movement of the arm (2) by:
∘ generating (805), if the calculated horizontal deviation is greater than a first threshold value, an instruction to horizontally move the arm (2) in the horizontal plane XY in order to reduce the deviation between the current horizontal position and the desired final horizontal position,
∘ generating (808), if the calculated horizontal deviation is less than the first threshold value and if the estimated vertical distance is greater than a second threshold value, an instruction to vertically move the arm (2) along a vertical direction in order to reduce the distance between the free end of the arm and the ocular tissue,
∘ generating (810), if the calculated horizontal deviation is less than the first threshold value and if the measured vertical distance is less than the second threshold value, an instruction to immobilize the arm.

8. The monitoring method according to claim 7, *wherein* the calculation step includes the following sub-steps:
- detecting, from the image of the current measurement pair, the horizontal position of at least one point of interest of the ocular tissue,
- evaluating, from the detected horizontal position of the point of interest, a horizontal deviation between:
∘ the current horizontal position of the free end of the arm in the horizontal plane XY, and
∘ the desired final horizontal position of the free end of the arm in the horizontal plane XY.

9. The monitoring method according to claim 8, *wherein* the detection sub-step consists in identifying the ocular tissue in the acquired image, by the implementation of a shape recognition algorithm to detect three concentric circles in the image.

10. The monitoring method according to any one of claims 7 to 9, *wherein* the therapy apparatus further comprises a force sensor (3) mounted on the free end of the arm (2) for measuring a mechanical force applied to the free end of the arm (2):
- the processing phase comprising a step of comparing said measured mechanical force with a third threshold value to determine whether the free end of the arm is in contact with an element that obstructs a vertical movement of the arm along the Z axis,
- the servo-control step including the generation of an instruction to immobilize the arm if the measured mechanical force is greater than the third threshold value.

11. The monitoring method according to any one of claims 7 to 10, *wherein* the acquisition phase comprises:
- the acquisition, by laser ranging, of a signal representative of a vertical distance along the Z axis, and/or
- the acquisition, by ultrasounds, of a signal representative of a vertical distance along the Z axis, and/or,
- the extraction of an acquired image from a signal representative of a vertical distance along the Z axis.

12. The monitoring method according to any one of claims 7 to 11, *wherein* the servo-control step includes:
- generating an elementary movement instruction to allow the movement of the arm between its current position and a desired final position,
- generating an immobilization instruction subsequent to each elementary movement instruction,
- repeating the previous sub-steps until the calculated horizontal deviation is less than the first threshold value and the measured vertical distance is less than the second threshold value.
